# EUROPEAN PATENT APPLICATION

(11) **EP 4 513 447 A1**
(43) Date of publication of application: **26.02.2025**
(21) Application number: 24195992.3
(22) Date of filing: 22.08.2024
(51) Int. Cl.: G06V 10/82

(54) **METHOD AND APPARATUS FOR CHECKING A KIT OF SURGICAL INSTRUMENTS**

(30) Priority: 24.08.2023 IT 202300017547
(71) Applicant: PROMEDITAL S.R.L., 42122 Reggio nell'Emilia (RE) (IT)
(72) Inventor: Tamarozzi, Marco, Modena (Mo) (IT)
(74) Representative: Petraz, Davide Luigi

(57) **Abstract**

Method and apparatus for checking a kit of surgical instruments (150) contained inside a container (151) by means of an optical detection device (25) that acquires at least one image of the plurality of instruments (150), wherein it is provided to check whether all the instruments (150) provided in the kit are present, and whether they are in the intended position inside the container (151) by comparing the acquired image with reference images contained in a recognition engine stored in a storage module (42) of a control and management unit (40), process the image acquired through a processing unit (41) of the control and management unit (40) to obtain the images of the single instruments (150) and detect their outline, and recognize the single instruments by comparing the images processed with said reference images, wherein the recognition has a positive outcome in the event that it can be at least partly overlapped with one of said reference images with a degree of confidence higher than a certain default threshold and display on a user interface (I) the outcome of the checking and recognition steps.

## Description

### FIELD OF THE INVENTION

The present invention relates to a method for checking a kit of surgical instruments and to a related apparatus suitable for carrying out the aforementioned method.

### BACKGROUND OF THE INVENTION

In the health sector, for example in hospitals, or in general in any facility where operating rooms suitable for surgical interventions are provided, it is necessary to manage kits of surgical instruments, for example, surgical instruments such as scalpels, retractors, scissors and the like, but also gauzes, swabs, gauze swabs and the like.

These kits of instruments can be, for example, single-surgery procedural kits, each dedicated to a particular type of surgical intervention.

Currently, a nurse instrumentalist performs an instrument count at the beginning and end of the intervention and only they are aware of the exact composition of the kit.

At the end of each intervention, a reconditioning procedure is initiated for the instruments comprised in the kit, in which the instruments are washed, sterilised and possibly checked and maintained.

In addition, the kit must be reconstituted by a specialized operator who checks the list of necessary instruments, picks them up and places them in a container for later use.

These operations are time-consuming, as many kits can arrive from the operating rooms requiring a high degree of attention and concentration from the operator in order to do everything accurately and reliably.

It should be noted that there are various types of kits consisting of a large number of instruments, up to 150/200 instruments, possibly repeated within the same kit, or in which there are instruments similar in shape to each other that differ in minute details, such as a slight scale factor for certain parts of the instrument, so that many instruments are easily confused with each other.

In some cases, kits of surgical instruments can be loaned for use, for example in the case of special kits dedicated to a particular intervention and containing particularly expensive surgical instruments, usually for orthopaedic interventions. For these kits, usually rented by specialised companies, it is usually necessary to carry out at least one check when sending the kit and one check when returning it, to verify that it is returned complete.

The possibility of making mistakes in the kit reconstitution operations is therefore very high, although this operation is generally carried out by trained and highly specialized personnel.

The need to optimise the process of recognizing instruments, certifying that they belong to kits, recognizing the absence or defectiveness of an instrument within a kit for reassembling a kit of surgical instruments before or after use, verifying that it is complete, is therefore very apparent in the hospital environment.

In the state of the art, methods of identifying instruments by placing serial codes on the instrument are known, such as engraving barcodes, such as qrcodes or datamatrix, for example by laser or micro-engraving.

A disadvantage of such methods is their invasiveness, as they provide for engraving the code directly on the surface of the instrument itself, which entails numerous drawbacks including the likelihood of compromising the compliance of the instrument with the requirements necessary to qualify as a medical device, of decreasing its safety of use, as dirt may accumulate or surface defects, such as rust, may arise in the area where the engraving takes place, or the fact that the engraving may at least partially fade with time.

Alternative methods, known in the art, provide for the affixing of RFID (Radio Frequency IDentification) devices in the instrument.

These methods, in addition to being subject to the same drawbacks listed above, also have the additional drawback that the tag could be detached from the instrument, even during surgery.

In addition, the known marking steps are a complex and expensive operation, usually carried out outside the hospital setting.

In European Patent Application EP 4022499 A1, the Applicant describes an apparatus, and a related method, for the recomposition of an kit of instruments comprising a support device provided with a backlit rest plane for the instruments, at least one optical detection device configured to acquire an image of the plurality of instruments arranged on the rest plane, a data processing system adapted to acquire data from the optical detection devices and process them to recognize the instruments by comparing the acquired image with reference images. This solution, which offers an effective and reliable method of recomposing kits of instruments, can however be improved from the point of view of automatic recognition of the instruments belonging to the kit.

There is therefore the need to perfect a method for checking a kit of instruments, particularly surgical instruments, and a related apparatus that can overcome at least one of the disadvantages of the state of the prior art.

To do this, it is necessary to solve the technical problem of defining a hardware and software architecture with high reliability for an apparatus and a method for checking a kit of surgical instruments arranged in an orderly manner inside a suitable container.

In particular, one purpose of the present invention is to provide an apparatus for checking kits of surgical instruments, and to develop a related method, which allows human error to be minimized and allows the recomposition of the kit to be made faster and easier.

One purpose of the present invention is also to provide an apparatus and a method capable of recognizing instruments with a high degree of reliability, for example with an accuracy of more than 80%.

Another purpose is to provide a non-invasive method, which avoids engraving or marking of the instruments.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, and to resolve the technical problem disclosed above in a new and original manner, also achieving considerable advantages compared to the state of the prior art, a method for checking a kit of surgical instruments contained inside a container comprises the steps of:
- making available a recognition engine trained by means of reference images of said instruments and said kits and stored in a storage module of a control and management unit;
- acquiring at least one image which overall depicts said plurality of instruments by means of an optical detection device;
- checking by means of a processing unit of said control and management unit whether all the instruments provided in the kit are present, and if they are in the intended position inside the container, comparing the acquired image with said reference images of said kit;
- processing said image through said processing unit to obtain the images of the single instruments and detect their outline;
- recognizing by means of said processing unit the single instruments by comparing said processed images with the reference images in said recognition engine for the recognition of said single instrument, wherein said recognition has a positive outcome in the event that it can be at least partially overlapped on one of said reference images with a degree of confidence higher than a certain predefined threshold;
- displaying on a user interface the outcome of said checking step and said recognition step.

In accordance with another aspect of the present invention, the method may comprise a preliminary training step for the creation of the aforementioned recognition engine. For training, the method may include, for each surgical instrument comprised in an operating kit, acquiring an image of a single instrument at a time.

In accordance with another aspect of the present invention, the method may include using, as a recognition engine, a neural network.

The aforementioned training step may therefore include dividing the set of images of the instruments acquired into a training set and a validation set.

In accordance with another aspect of the present invention, the step of processing the image of a single instrument comprises the use of HDR software, after acquiring multiple images with different exposures, so that each detail is correctly exposed in at least one of the images.

In accordance with another aspect of the present invention, the step of processing the image of a single instrument comprises processing by means of software selected from software comprising medium filters, adaptive thresholds and canny edge detection software.

In accordance with another aspect of the present invention, the step of processing the image of a single instrument may comprise processing the image of the single instrument by means of software configured to perform morphological dilation and/or erosion operations to define an outline of the instrument.

In accordance with another aspect of the present invention, an apparatus for checking a kit of surgical instruments contained inside a container comprises an optical detection device configured to detect an image of the kit of said instruments and a user interface for displaying the outcome of the check, and further comprises a control and management unit comprising a processing unit and a storage module and operatively connected to said optical detection device and to said interface. The storage module is configured as a recognition engine in which images of the kits of instruments to be checked are stored. Said processing unit is configured to check whether all the instruments provided in the kit are present, and whether they are in the intended position inside the container by comparing the acquired image with said reference images of said kit and is also configured to process the acquired image so as to obtain the images of the single instruments and detect their outline and to recognize the single instruments by comparing said processed images with the images stored in said recognition engine.

In accordance with one aspect of the present invention, the apparatus comprises a work chamber delimited at least by a base wall configured to receive said container restingly, by an upper wall parallel to said base wall, to which said optical detection device is associated, and by a connecting wall, which connects said base and upper walls and is perpendicular to the latter.

In accordance with one aspect of the present invention, the apparatus further comprises a pair of side walls connected to both said base wall and said upper wall and said connecting wall, which is configured as a rear wall with respect to said work chamber.

In accordance with one aspect of the present invention, the apparatus comprises a front wall, parallel to said connecting wall and of lower extension than the latter to allow an operator to access said work chamber.

The method and apparatus according to the present invention advantageously allow a kit of surgical instruments to be checked effectively and reliably.

An advantage of the method and apparatus according to the present invention is to be able to automatically check whether the kit of surgical instruments contains all the instruments intended, whether they are all in the intended position inside the container and whether the instruments present are the correct ones.

The method and the apparatus according to the present invention significantly facilitate the operators, to whom it is communicated through the interface, in an intuitive way, whether the checked kit is complete and correct.

The method and apparatus according to the present invention can be advantageously used in cases wherein the kits of surgical instruments are delivered on loan for use to the hospital or clinic facility that is to use them. In fact, this way of managing the kits involves checking the kits several times, for example at the time of arrival at the facility of use, and after use, before returning, significantly speeding up the checking operations of the kits of surgical instruments.

An advantage of the apparatus according to the present invention is that it is compact and easily transportable.

An advantage of the apparatus according to the present invention is to have a configuration in which the work chamber is delimited by several walls so as to optimize the viewing of the instruments by the optical recognition device and make the recognition more effective.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a perspective, schematic view illustrating an apparatus for checking a kit of surgical instruments according to the present invention;
- fig. 2 is a perspective, schematic and partially exploded view, illustrating an example of a kit of surgical instruments placed inside their container;
- fig. 3 is a schematic and perspective top view of a variant embodiment of the container of Fig. 2, in which some surgical instruments are also visible;
- figures 4 and 5 are schematic, perspective views illustrating further embodiments of an apparatus for checking a kit of surgical instruments according to the present invention.

We must clarify that in the present description the phraseology and terminology used, as well as the figures in the attached drawings also as described, have the sole function of better illustrating and explaining the present invention, their function being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS OF THE PRESENT INVENTION

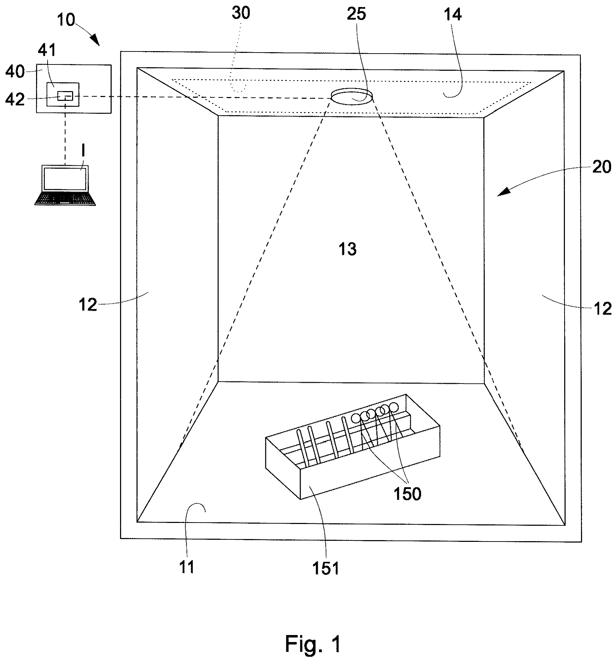

With reference to figures 1-5, an apparatus 10 for checking kits of surgical instruments 150 according to the present invention is described, wherein the instruments 150 are arranged in an orderly manner inside a container 151.

The container 151 may be reclosed using a lid 152.

Furthermore, the container 151 may comprise a plurality of internal partition walls, 153 configured to compartmentalize the space between the instruments 150 and prevent them from moving freely inside the container.

Each container 151 is associated with a recognition code 158, for example a bar code, by reading which it is possible to know which are the instruments 150 of the kit contained therein.

In embodiments, the container 150 is made of metal material delimited by side and bottom walls shaped like a grid so that it can also be subjected to the washing and sterilization treatments to which the instruments 150 are also subjected.

In accordance with a first embodiment, see fig. 1, the apparatus 10 has a box-like shape, for example a substantially cubic shape. In other embodiments, the apparatus has the shape of a cube or of a parallelepiped with a square or rectangular base.

The apparatus 10 comprises a work chamber 20, configured to receive the container 151.

The work chamber 20 comprises a base wall 11 that delimits it at the bottom defining a rest plane for the container 151.

The apparatus further comprises a pair of side walls 12 and a rear wall 13, all three connected to the base wall 11 so as to delimit the work chamber 20 on three sides.

The work chamber 20 comprises an upper wall 14, parallel to the base wall 11 to delimit said work chamber 20 at the top, and connected to both the side walls 12 and the rear wall 13.

In the example provided herein, said walls define the faces of a cube, wherein the front wall is missing to allow operators to access the base wall 11 to rest or pick up the container 150.

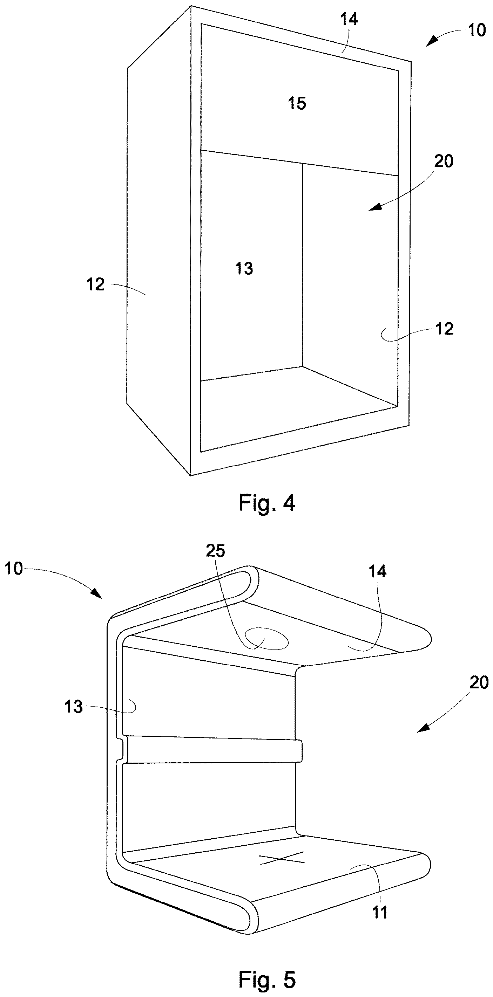

In another embodiment according to the present invention, see fig. 4, a front wall 15 is provided, connected to said upper wall 14, and parallel to the rear wall 13, but having a lower extension than the latter to define a front opening that allows operators to access the base wall 11.

In a further embodiment according to the present invention, see fig. 5, the apparatus 10 is devoid of the side walls 12. In this case, the work chamber 20 is delimited at the bottom by the base wall 11, at the rear by the rear wall 13 and at the top by the upper wall 14.

In other versions of the apparatus 10 according to the present invention, not shown, a front wall can be provided, parallel and having the same extension as the rear one, which is openable and closable if necessary, so that the work chamber 20 can be completely closed, for example when not in use and/or when it is necessary to transport the apparatus. This front wall can be configured as a hinged door on one of the adjacent side walls.

The apparatus comprises an optical detection device 25 associated with the upper wall 14, preferably positioned substantially in the centre of said wall so that the entire base wall 11 falls completely within its field of view.

The optical detection device 25 is configured to acquire images of the instruments 150 arranged inside the container 151.

The optical detection device 25 may comprise a camera with high resolution, preferably greater than 16.2 MP.

Advantageously, this optical detection device 25 allows obtaining a high resolution per millimetre and therefore recognizing instruments that differ by a few millimetres.

The optical detection device 25 can be integrated in the upper wall 14 or can protrude from the latter downwards, i.e. towards the base wall 11.

In other embodiments, not shown, the apparatus 10 comprises further optical detection devices, which can also be placed on the upper wall 14 or on the rear wall 13 and/or on the side walls 12.

The apparatus 10 also comprises a lighting unit 30, also associated with the upper wall 14, to illuminate the base wall 11 on which the container 151 is arranged restingly.

The lighting unit 30 comprises a lamp, for example an LED lamp, configured to illuminate the base wall by light radiation that is preferably homogeneous over the entire rest plane.

In embodiments, the upper lamp may be configured to emit a flash. The illumination time interval can in this case be comprised between one millisecond and 500 milliseconds.

In such embodiments, the lamp may adjust the intensity of the aforementioned light radiation between at least a first and a second predetermined level of light intensity. During normal operation, the intensity of the lamp can be maintained at said first light intensity level, while when the lamp emits a flash, it emits at the second predetermined light intensity level.

The apparatus 10 further comprises a user interface I for interaction with an operator and comprises instruction acquisition devices and display devices.

The instruction acquisition devices can be devices that allow a contactless interaction with the operator, for example an audio device able to acquire vocal instructions such as a microphone, and/or a touch screen, and/or a keyboard and/or the like.

The display devices may be one or more monitors, a tablet screen, a smartphone, a notepad, and/or the like.

Preferably, in one embodiment, the user interface I comprises augmented reality means, such as a headset intended to be worn by the operator and implementing augmented reality techniques. In this case, the user interface I lacks a physical screen but is provided with a virtual screen, visible to the operator wearing the headset, suitable for being configured as a display means.

The aforementioned display devices and/or augmented reality means may be configured to display the outcome of the check of the kit of instruments 150.

The augmented reality means can integrate devices for checking the apparatus through gestures (gesture control), partially or completely replacing the aforementioned instruction acquisition devices.

Advantageously, the use of the aforementioned augmented reality means decreases the need to integrate complex and bulky hardware components into the apparatus 10, such as servers, personal computers, monitors and/or keyboards. In this way, the apparatus 10 may also be suitable for environments where available space is limited, as well as facilitating communication with the operator. In fact, in this embodiment it is no longer necessary to have monitors and touch screens, since the augmented reality means can project all the useful information in front of the operator.

The apparatus 10 comprises a control and management unit 40 comprising a processing unit 41 and a storage module 42.

The control and management unit 40 is operatively connected both to the optical detection device 25, from which it receives the information relating to the detected images, and to the user interface I to which it sends the information to be made available to the user or from which it acquires input information entered by the user.

The control and management unit 40 is also operatively connected to the lighting unit 30 to control its operation.

The processing unit 41 is configured to process an image detected by the optical detection device 25 wherein the image comprises the entire kit of instruments 150.

The processing unit 41 communicates with the storage module 42 which is configured as a recognition engine in which images of the kits of instruments 150 to be checked are stored, in a dedicated database.

The recognition engine may be trained by means of reference images of the instruments 150. The recognition engine may implement algorithms based on Artificial Intelligence and Machine Learning, for example a neural network, an SVM (Support Vector Machine) algorithm, a genetic algorithm or the like.

The recognition engine can be trained in a known manner by means of a set of images of the instruments, for example divided into a training set and a validation set.

The recognition engine can be configured to recognize the image of a single instrument 150 in the event that it can be at least partly overlapped with one of the reference images comprised in said database.

It may be provided that the recognition takes place with a degree of confidence higher than a certain predefined threshold.

For these purposes, the processing unit 41 may implement an image processing algorithm.

To compare images acquired at various exposure levels, the algorithm may comprise the use of HDR software.

For comparison, the algorithm may process the images by means of software selected from software comprising median filters, adaptive thresholds, canny edge detection software, and the like.

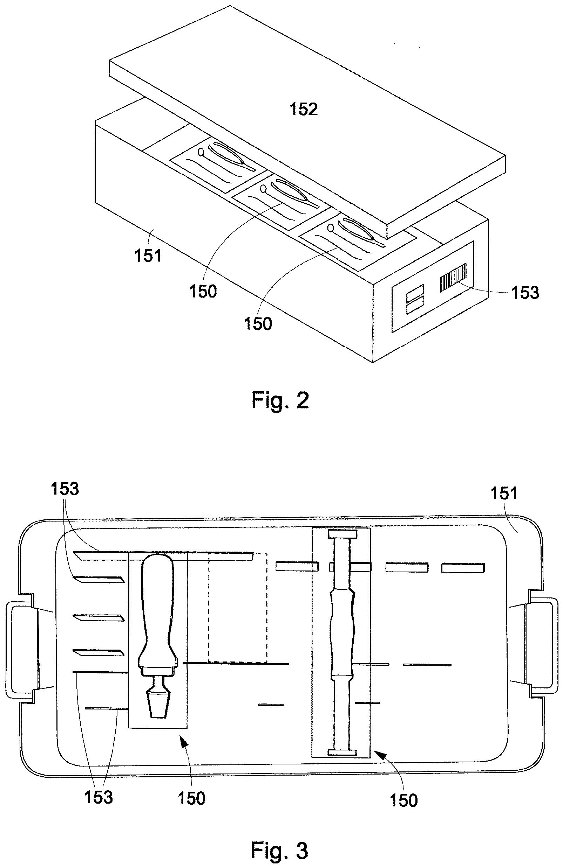

The algorithm may be configured to process the images by deriving images representing a single instrument 150, in a segmentation operation. It may then be configured to process the image of the single instrument 150 by means of software configured to perform morphological dilation and/or erosion operations to define an outline of the instrument 150, as exemplified in fig. 3.

The algorithm may be configured to detect parameters characteristic of the shape and size of each instrument.

The algorithm may be configured to repeat the processing for each instrument comprised in the detected image of the kit of instruments by the optical detection device 25. The comparison may then be conducted on each image of the single instrument 150.

In embodiments, the algorithm also compares the acquired image with reference images which depict the entire kit of instruments 150 in order to be able to verify whether each instrument is in the intended location.

The processing unit 41 may implement a recognition algorithm configured to compare the image detected by the optical detection device 25 and subsequently processed by the image processing algorithm with reference images of the instruments 150.

The reference images may be contained in a storage module 42, comprised in the processing unit 41 and/or in the control and management unit 40.

The recognition algorithm may be configured to provide a positive outcome of the recognition of the instruments 150 in the event that the compared image can be overlapped with a good degree of confidence on the corresponding reference image. For example, the outcome is positive if the overlap is higher than a predefined threshold equal to 80%.

The operation of the apparatus 10 described herein, which corresponds to the method according to the present invention, comprises arranging a container 151 containing a kit of surgical instruments 150 on the base wall 11, after removing the lid 152.

Before or during this step, the method involves recognizing which kit it is by reading, by means of a suitable reader comprised in the apparatus, an identification code 153, for example a bar code, affixed to the container 151.

At this point the processing unit 41 recognizes which kit it is and consequently which instruments 150 should be part of it.

The method then provides for a step of acquiring an image which overall depicts the plurality of instruments 150 by means of the optical detection device 12. During the acquisition of the image, a simultaneous step of illuminating the base wall 11 by means of light radiation, for a predetermined time interval, is provided.

The method comprises a preliminary checking step in which it verifies both whether all the instruments 150 of the kit are present, and whether each of them is placed in the intended position. To perform this preliminary checking step, the processing unit 41 compares the image acquired in the previous acquisition step with a reference image of the kit in question stored in the storage module 42. In the example illustrated in Fig. 3, the position where an instrument 150 is missing is indicated with a dashed rectangle, which information will then be made available to the operator through the interface I.

Subsequently, the method provides for a processing step in which the processing unit 41 processes the image acquired by means of an image processing algorithm, after having segmented it so as to isolate each single instrument. Image processing involves employing suitable image processing software to detect the outline of the instrument 150.

Thereafter, the method provides for a recognition step in which the processing unit 41 implements a recognition algorithm during which the recognition engine trained by means of reference images of the instruments 150 and stored in the storage unit 42 compares the image processed in the previous processing step with the stored images.

Advantageously, during the recognition step it is provided to compare the processed image only with the stored images of the instruments that must be comprised in the kit in question and not with all the images in the database, in order to speed up the comparison.

The recognition step has a positive outcome in the event that the processed image can be at least partially overlapped with one of the reference images with a degree of confidence higher than a certain predefined threshold.

It is noted that the recognition algorithm allows the instruments 150 to be effectively recognized even in the case where the bottom wall and the side walls are configured as metal grids.

The method then comprises a step of displaying the outcome of the recognition, wherein it is displayed on a user interface I.

The training of the recognition engine can be performed before the use of the apparatus 10, for example during the installation step. In the case that an image database suitable for the kit(s) of instruments 150 to be recognized is already available, the initial training step can be avoided.

The preliminary training step may provide that, for each surgical instrument 150 comprised in an operating kit, an image of a single instrument 150 at a time is acquired by means of the optical recognition device 25.

Advantageously, the recognition algorithm according to the invention is able to recognize, and then classify, hundreds of different instruments that also differ by 1-2 mm, under varying environmental conditions and with an accuracy of more than 80%.

It is clear that modifications and/or additions of parts and/or steps can be made to the apparatus 10 and to the method as described heretofore, without departing from the field and scope of the present invention, as defined by the claims.

It is also clear that, although the present invention has been described with reference to some specific examples, a person of skill in the art shall certainly be able to achieve other equivalent forms of the method for recognition of surgical instruments and the related apparatus, having the characteristics as set forth in the claims and hence all coming within the field of protection defined thereby.

In the following claims, the sole purpose of the references in brackets is to facilitate their reading and they must not be considered as restrictive factors with regard to the field of protection defined by the claims.

## Claims

1. Method for checking a kit of surgical instruments (150) contained inside a container (151), wherein the method is **characterized in that** it comprises the steps of
- making available a recognition engine trained by means of reference images of said instruments (150) and said kits and stored in a storage module (42) of a control and management unit (40);
- acquiring at least one image which overall depicts said plurality of instruments (150) by means of an optical detection device (25);
- checking by means of a processing unit (41) of said control and management unit (40) whether all the instruments (150) provided in the kit are present, and whether they are in the intended position inside the container (151) by comparing the acquired image with said reference images of said kit;
- processing said image by means of said processing unit (41) to obtain the images of the single instruments (150) and detect their shape;
- recognizing by means of said processing unit (41) the single instruments (150) by comparing said processed images with the reference images in said recognition engine for the recognition of said single instrument (150), wherein said recognition has a positive outcome in the event that it can be at least partly overlapped with one of said reference images with a degree of confidence higher than a certain predefined threshold;
- displaying on a user interface (I) the outcome of said checking step and said recognition step.

2. Method as in claim 1, **characterized in that** it comprises a preliminary training step for the creation of said recognition engine which, for each surgical instrument (150) comprised in an operating kit, provides to acquire an image of a single instrument (150) at a time.

3. Method as in claim 1 or 2, **characterized in that** it provides to use a neural network as a recognition engine.

4. Method as in any claim hereinbefore, **characterized in that** said step of processing the image of a single instrument (150) comprises processing by means of software selected from HDR (High Dynamic Range) software, software comprising median filters, adaptive thresholds, canny edge detection software and/or software configured to perform morphological dilation and/or erosion operations to define an outline of the instrument (150).

5. Method as in any claim hereinbefore, **characterized in that** it comprises illuminating said base wall (11) by means of a light radiation, for a predetermined time interval during said step of acquiring said image.

6. Apparatus (10) for checking a kit of surgical instruments (150) contained inside a container (151), wherein the apparatus (10) comprises an optical detection device (25) configured to detect an image of the kit of said instruments (150) and a user interface (I) for displaying the outcome of the check, **characterized in that** it comprises a control and management unit (40) comprising a processing unit (41) and a storage module (42) and operatively connected to said optical detection device (25) and said interface (I) of the apparatus, wherein said storage module (42) is configured as a recognition engine in which images of the kits of instruments (150) to be checked are stored and wherein said processing unit (41) is configured to check whether all the instruments (150) provided in the kit are present, and whether they are in the intended position inside the container (151) by comparing the acquired image with said reference images of said kit and is also configured to process the acquired image so as to obtain the images of the single instruments (150) and detect their outline and to recognize the single instruments (150) by comparing said processed images with the images stored in said recognition engine.

7. Apparatus (10) as in claim 6, **characterized in that** it comprises a work chamber (20) delimited at least by a base wall (11) configured to receive said container (151) restingly, by an upper wall (14) parallel to said base wall (11), to which said optical detection device (25) is associated, and by a connecting wall (13), which connects said base (11) and upper (14) walls and is perpendicular to the latter.

8. Apparatus (10) as in claim 7, **characterized in that** it further comprises a pair of side walls (12) connected both to said base wall (11) and to said upper wall (14) and to said connecting wall (13) which is configured as a rear wall with respect to said work chamber (20).

9. Apparatus (10) as in claim 7 or 8, **characterized in that** it comprises a front wall (15), parallel to said connecting wall (13) and of lower extension than the latter to allow an operator to access said work chamber (20).

10. Apparatus (10) as in claims 8 or 9, **characterized in that** it comprises further optical detection devices (25) also associated with said connecting wall (13) and/or with said pair of side walls (12).

11. Apparatus (10) as in any claim from 6 to 10, **characterized in that** it is box-shaped, in particular the shape of a cube or of a parallelepiped with a square or rectangular base.

12. Apparatus (10) as in any claim from 6 to 11, **characterized in that** it comprises a lighting unit (30) associated with said upper wall (14) to illuminate said base wall (11) and comprising a LED lamp, configured to illuminate the base wall (11) by means of homogeneous light radiation.

13. Apparatus (10) as in any claim from 6 to 12, **characterized in that** said interface (I) comprises augmented reality means intended to be worn by an operator which are configured to display information and receive instructions from said operator.

14. Apparatus (10) as in any claim from 6 to 13, **characterized in that** said interface (I) comprises an audio device able to acquire vocal instructions from said operator.
